# EUROPEAN PATENT APPLICATION

(11) **EP 4 316 480 A1**
(43) Date of publication of application: **07.02.2024**
(21) Application number: 22775267.2
(22) Date of filing: 15.03.2022
(51) Int. Cl.: A61K 31/197, A61K 31/704, A61K 33/26, A61P 35/00, A61P 43/00

(54) **PROPHYLACTIC AGENT OR THERAPEUTIC AGENT FOR SIDE EFFECTS OF ANTHRACYCLINE ANTICANCER AGENT**

(30) Priority: 23.03.2021 JP 2021048209
(71) Applicant: Kyushu University, National University Corporation, Nishi-ku Fukuoka-shi Fukuoka 819-0395 (JP)
(72) Inventor: IKEDA, Masataka, Fukuoka-shi, Fukuoka 819-0395 (JP); IDE, Tomomi, Fukuoka-shi, Fukuoka 819-0395 (JP); TSUTSUI, Hiroyuki, Fukuoka-shi, Fukuoka 819-0395 (JP); ABE, Kou, Fukuoka-shi, Fukuoka 819-0395 (JP)
(74) Representative: Weickmann & Weickmann PartmbB
(86) International application number: PCT/JP2022/011540
(87) International publication number: WO 2022/202477

(57) **Abstract**

Provided is a widely applicable and safe prophylactic agent or therapeutic agent for adverse reaction of an anthracycline anticancer agent. In cancer treatment with an anthracycline anticancer agent, 5-aminolevulinic acids are used in combination therewith.

## Description

### [Technical Field]

The present invention relates to a pharmaceutical composition for preventing or treating adverse reaction of an anthracycline anticancer agent.

### [Background Art]

Anthracycline anticancer agents including doxorubicin are used in the treatment of many cancers including leukemia, lymphoma, breast cancer, uterus cancer, ovary cancer, and lung cancer. The anthracycline anticancer agents tend to manifest adverse reaction in a dose-dependent manner and induce irreversible disorder of myocardial contraction called anthracycline (doxorubicin) cardiomyopathy at more than given doses, and life prognosis after development thereof is very poor. Hence, cumulative doses of the anthracycline anticancer agents are strictly restricted (500 mg/m² or less for doxorubicin) under practice guidelines. However, particularly, blood tumor, which repeats remission and recurrence, not infrequently leads to such dose limits, and this limitation causes the therapy regimen as first-line choice to be given up. Under these circumstances, chemotherapy for malignant tumor is insufficient. In spite of the strict dose limitation, cardiotoxicity occurs in a little under 20% of patients given the anthracycline anticancer agents, and in a great majority thereof, cardiotoxicity reportedly occurs within 1 year from the completion of chemotherapy. Thus, the establishment of a method for preventing anthracycline (doxorubicin) cardiomyopathy is indispensable for performing sufficient chemotherapy for malignant tumor without developing cardiomyopathy. However, the mechanism of manifestation of cardiotoxicity has not yet been clearly elucidated, and any effective coping method has not yet been established.

Dexrazoxane is known as a therapeutic drug for extravasation in the intravenous administration of an anthracycline anticancer agent (Non Patent Literature 1).

### [Citation List]

### [Non Patent Literature]

[Non Patent Literature 1] Langer S.W. Dexrazoxane for the treatment of chemotherapy-related side effects. Cancer Manag. Res. 2014; 6: 357-363

### [Summary of Invention]

### [Technical Problem]

As described above, dexrazoxane is known as a therapeutic drug for adverse reaction of an anthracycline anticancer agent and however, is limited by extravasation as an applicable situation in Japan. Although dexrazoxane has been approved as a prophylactic drug for doxorubicin cardiomyopathy overseas centered on the USA, there are growing concerns about the attenuation of an antitumor effect of doxorubicin by dexrazoxane. Furthermore, not only is dexrazoxane itself also found to have adverse reaction such as myelosuppression, nausea, fever or pain at injection sites, and vomiting, but the possibility of inducing secondary malignant tumor has been reported. Under these circumstances, use of dexrazoxane itself is strictly restricted by FDA. Thus, there is a strong demand for the development of a drug suppressing the emergence of cardiotoxicity ascribable to an anthracycline anticancer agent, and safe use of the anticancer agent by combined use thereof. Hence, it is desired to develop a more widely applicable and safe prophylactic agent or therapeutic agent for adverse reaction of anticancer agents.

### [Solution to Problem]

The present inventors have found that, surprisingly, combined use of an anthracycline anticancer agent and 5-aminolevulinic acids (ALAs) can reduce adverse reaction while maintaining the drug efficacy of the anthracycline anticancer agent.

Specifically, in one embodiment, the present invention relates to a pharmaceutical composition for reducing toxicity of an anthracycline anticancer agent, comprising 5-aminolevulinic acids or derivatives thereof or salts of the acids or the derivatives as an active ingredient.

In one embodiment of the present invention, the pharmaceutical composition further comprises an iron compound.

In one embodiment of the present invention, the toxicity is depression of cardiac functions or elevation in lipid peroxide level.

In one embodiment of the present invention, the pharmaceutical composition is orally or transvenously administered.

In one embodiment of the present invention, the pharmaceutical composition is prepared such that the 5-aminolevulinic acids or the derivatives thereof or the salts of the acids or the derivatives are administered at a dose of 0.5 mg/kg to 20 mg/kg to the subject.

In one embodiment of the present invention, the pharmaceutical composition contains 5 mg to 1500 mg of the 5-aminolevulinic acids or the derivatives thereof or the salts of the acids or the derivatives.

In one embodiment of the present invention, the pharmaceutical composition is administered to the subject concurrently with administration of the anthracycline anticancer agent or 2 hours to 7 days before the administration.

In one embodiment of the present invention, the pharmaceutical composition is administered every day to the subject over a period from 1 day to 15 days before administration of the anthracycline anticancer agent.

In one embodiment of the present invention, the pharmaceutical composition is continuously administered to the subject from at least 3 days (preferably 7 days) before administration of the anthracycline anticancer agent to at least 7 days (preferably 14 days) after the administration of the anthracycline anticancer agent.

In one embodiment of the present invention, the administration cycle is repeated every 1 week to 4 weeks.

In one embodiment of the present invention, the 5-aminolevulinic acids are compounds represented by the following formula (I):
[Formula 1]

R¹-NHCH₂COCH₂CH₂COOR² (I)

wherein R¹ represents a hydrogen atom or an acyl group, and R² represents a hydrogen atom, a linear or branched alkyl group, a cycloalkyl group, an aryl group, or an aralkyl group
or pharmacologically acceptable salts or esters thereof.

In one embodiment of the present invention, the iron compound is one or two or more iron compounds selected from the group consisting of ferrous citrate, sodium ferrous citrate, sodium iron citrate, ammonium iron citrate, ferric pyrophosphate, heme iron, dextran iron, iron lactate, ferrous gluconate, DTPA iron, sodium iron diethylenetriaminepentaacetate, ammonium iron diethylenetriaminepentaacetate, sodium iron ethylenediaminetetraacetate, ammonium iron ethylenediaminepentaacetate, triethylenetetramine iron, sodium iron dicarboxymethylglutamate, ammonium iron ammonium dicarboxymethylglutamate, lactoferrin iron, transferrin iron, ferric chloride, iron sesquioxide, sodium iron chlorophyllin, ferritin iron, ferrous fumarate, ferrous pyrophosphate, saccharated iron oxide, iron acetate, iron oxalate, ferrous succinate, sodium iron citrate succinate, iron sulfate, and iron glycine sulfide.

In one embodiment of the present invention, the anthracycline anticancer agent is an anticancer agent selected from the group consisting of doxorubicin, aclarubicin, pirarubicin, idarubicin, epirubicin, daunorubicin, amrubicin, their derivatives, and their pharmacologically acceptable salts.

An alternative embodiment of the present invention relates to a pharmaceutical composition for suppressing tumor growth, comprising an anthracycline anticancer agent as an active ingredient, wherein the pharmaceutical composition is used with 5-aminolevulinic acids or derivatives thereof or salts of the acids or the derivatives.

In one embodiment of the present invention, the 5-aminolevulinic acids or the derivatives thereof or the salts of the acids or the derivatives are administered with an iron compound to the subject.

In one embodiment of the present invention, the 5-aminolevulinic acids or the derivatives thereof or the salts of the acids or the derivatives are administered for reducing toxicity of the anthracycline anticancer agent.

In one embodiment of the present invention, the toxicity is depression of cardiac functions or elevation in lipid peroxide level.

In one embodiment of the present invention, the 5-aminolevulinic acids or the derivatives thereof or the salts of the acids or the derivatives are orally administered.

In one embodiment of the present invention, a therapeutically effective amount of the anthracycline anticancer agent is administered in combination with 0.5 mg/kg to 20 mg/kg of the 5-aminolevulinic acids or the derivatives thereof or the salts of the acids or the derivatives.

In one embodiment of the present invention, the 5-aminolevulinic acids or the derivatives thereof or the salts of the acids or the derivatives are administered concurrently with administration of the anthracycline anticancer agent or 2 hours to 7 days before the administration.

In one embodiment of the present invention, the 5-aminolevulinic acids or the derivatives thereof or the salts of the acids or the derivatives are administered every day over a period from 1 day to 15 days before (preferably 1 day to 3 days before) administration of the anthracycline anticancer agent.

In one embodiment of the present invention, the 5-aminolevulinic acids or the derivatives thereof or the salts of the acids or the derivatives are continuously administered to the subject from at least 3 days (preferably 7 days) before administration of the anthracycline anticancer agent to at least 7 days (preferably 14 days) after the administration of the anthracycline anticancer agent.

In one embodiment of the present invention, the administration cycle is repeated every 1 week to 4 weeks.

An alternative embodiment of the present invention relates to a method for reducing toxicity of an anthracycline anticancer agent in a subject, comprising the step of administering 5-aminolevulinic acids or derivatives thereof or salts of the acids or the derivatives to the subject concurrently with or separately from administration of the anthracycline anticancer agent to the subject.

An alternative embodiment of the present invention relates to a method for suppressing tumor growth in a subject, comprising the step of administering 5-aminolevulinic acids or derivatives thereof or salts of the acids or the derivatives and an anthracycline anticancer agent concurrently or separately to the subject.

Embodiments composed of one or two or more in combination of the features of the present invention described above are also included in the scope of the present invention.

### [Advantageous Effects of Invention]

5-Aminolevulinic acid (ALA) is a natural amino acid that is used as a precursor of heme *in vivo,* and is also used for various purposes such as medicaments and supplements. 5-Aminolevulinic acid is a substance confirmed to have high safety and as such, can be used as a prophylactic agent or a therapeutic agent for adverse reaction of an anthracycline anticancer agent in a wide range of subjects.

### [Brief Description of Drawings]

[Figure 1] Figure 1 shows that the administration of 5'-ALA suppressed increase in lipid peroxide level ascribable to the influence of doxorubicin.
[Figure 2] Figure 2 shows that the administration of 5'-ALA suppressed cell death ascribable to the influence of doxorubicin.
[Figure 3] Figure 3 shows that in an *in vivo* experimental system, the administration of 5'-ALA improved reduction in left ventricular systolic performance, which is indicated by left ventricular ejection fraction (LVEF), ascribable to the influence of doxorubicin.
[Figure 4] Figure 4 shows that in an *in vivo* experimental system, the administration of 5'-ALA suppressed increase in lipid peroxide (acrolein and MDA) level ascribable to the influence of doxorubicin.
[Figure 5] Figure 5 shows that the administration of 5'-ALA suppressed cell death ascribable to the influence of various anthracycline anticancer agents.
[Figure 6] Figure 6 shows that the level of ALAS1, a rate determining enzyme of a heme synthesis pathway in cardiomyocytes and cardiac muscular tissues, is decreased by the action of doxorubicin.
[Figure 7] Figure 7 shows that the level of protoporphyrin IX (PpIX), an intermediate metabolite of a heme synthesis pathway in cardiomyocytes, is decreased by the action of doxorubicin.
[Figure 8] Figure 8 shows that although iron excess and heme synthesis disorder occur in cardiomyocytes by the action of doxorubicin, these states are normalized by the action of 5'-ALA.
[Figure 9] Figure 9 shows that although iron excess and heme synthesis disorder occur in cardiac muscular tissues by the action of doxorubicin, these states are normalized by the action of 5'-ALA.
[Figure 10] Figure 10 shows that 5'-ALA does not attenuate a cell death-inducing effect which is the drug efficacy of doxorubicin.

### [Description of Embodiments]

The present invention is based on the finding that combined use of an anthracycline anticancer agent and 5-aminolevulinic acids (ALAs) is capable of reducing adverse reaction while maintaining the drug efficacy of the anthracycline anticancer agent. Specifically, the present invention relates to use of ALAs as a prophylactic agent or a therapeutic agent for adverse reaction of an anthracycline anticancer agent, or a combination of an anthracycline anticancer agent and ALAs. Alternatively, the present invention relates to use of ALAs in a standard therapy regimen using an anthracycline anticancer agent.

In the present disclosure, the anthracycline anticancer agent and the ALAs may be concurrently administered to a subject or may be separately administered thereto. Preferably, the ALAs are administered prior to administration of the anthracycline anticancer agent to the subject. In the case of administering the ALAs once, the ALAs may be administered to the subject, for example, concurrently with administration of the anthracycline anticancer agent to the subject, or at any timing of 2 hours to 7 days before the administration. In the present disclosure, the term "concurrently" means not strict simultaneity by minutes, but substantial simultaneity in consideration of a medical setting (e.g., the ALAs are administered within 2 hours before or after administration of the anthracycline anticancer agent). In the case of administering the ALAs a plurality of times, the ALAs may be continuously administered to the subject, for example, from at least 3 days before (preferably 7 days before) administration of the anthracycline anticancer agent. Also, the ALAs may be further administered to the subject over at least 7 days (preferably 14 days) after administration of the anthracycline anticancer agent. In the case of administering the anthracycline anticancer agent a plurality of times for cancer treatment, a treatment cycle with a combination of the anthracycline anticancer agent and the ALAs may be repetitively performed a plurality of times.

In general, the anthracycline anticancer agent mean a group of antitumor antibiotics derived from microbes of the genus *Streptomyces* and is also called anthracycline antibiotic. Typical examples of the anthracycline anticancer agent can include doxorubicin, aclarubicin, pirarubicin, idarubicin, epirubicin, daunorubicin, and amrubicin. The anthracycline anticancer agent according to the present invention is not limited thereto and can include all drugs that are generally classified as anthracycline anticancer agents. Furthermore, the scope of the present invention widely includes derivatives having the same or similar pharmacological activity as that of these compounds, preparations of these compounds, and the like.

The type of the cancer to which the present invention is to be applied is not limited and can be the type of a cancer for which the anthracycline anticancer agent has been confirmed so far to be effective. Non-limiting specific examples thereof can include malignant lymphoma, lung cancer, digestive system cancer (stomach cancer, gallbladder cancer, bile duct cancer, pancreatic cancer, hepatocellular cancer, large intestine cancer, etc.), bladder cancer, urothelial cancer, osteosarcoma, breast cancer, endometrial cancer, bone and soft tissue tumor, bone tumor, multiple myeloma, and children solid tumor (Ewing's sarcoma family of tumor, rhabdomyosarcoma, neuroblastoma, retinoblastoma, hepatoblastoma, nephroblastoma, etc.).

Cardiotoxicity (e.g., depression of cardiac functions, elevation in lipid peroxide level in cardiomyocytes or cardiac muscular tissues, and elevation in free iron level in mitochondria in cardiomyocytes or cardiac muscular tissues), myelosuppression, or the like is known as adverse reaction of the anthracycline anticancer agent. The present invention can be applied, particularly, to the prevention or treatment of cardiotoxicity.

The administration route of the anthracycline anticancer agent to the subject is not limited and can be, for example, an administration route (e.g., intravenous administration) that abides by instructions of a drug package insert. The dose of the anthracycline anticancer agent to the subject is not limited, and those skilled in the art (e.g., physicians) can determine the optimum dose according to the treatment stage, symptoms, etc. of the subject.

In the present specification, ALA means 5-aminolevulinic acid. ALA, also called δ-aminolevulinic acid, is a natural amino acid.

In the present invention, the derivative of ALA refers to a compound that is metabolized into PpIX *in vivo.* Examples thereof can include compounds represented by the formula (I) given below. In the formula (I), R¹ represents a hydrogen atom or an acyl group, and R² represents a hydrogen atom, a linear or branched alkyl group, a cycloalkyl group, an aryl group, or an aralkyl group. In the formula (I), ALA corresponds to the case where each of R¹ and R² is a hydrogen atom.
[Formula 2]

R¹-NHCH₂COCH₂CH₂COOR² (I)

Examples of the acyl group as R¹ in the formula (I) can include linear or branched alkanoyl groups having 1 to 8 carbon atoms, such as formyl, acetyl, propionyl, butyryl, isobutyryl, valeryl, isovaleryl, pivaloyl, hexanoyl, octanoyl, and benzylcarbonyl groups, and aroyl groups having 7 to 14 carbon atoms, such as benzoyl, 1-naphthoyl, and 2-naphthoyl groups.

Examples of the alkyl group as R² in the formula (I) can include linear or branched alkyl groups having 1 to 8 carbon atoms, such as methyl, ethyl, propyl, isopropyl, butyl, isobutyl, sec-butyl, tert-butyl, pentyl, isopentyl, neopentyl, hexyl, heptyl, and octyl groups.

Examples of the cycloalkyl group as R² in the formula (I) can include cycloalkyl groups having 3 to 8 carbon atoms which may be saturated or partially have an unsaturated bond, such as cyclopropyl, cyclobutyl, cyclopentyl, cyclohexyl, cycloheptyl, cyclooctyl, cyclododecyl, and 1-cyclohexenyl groups.

Examples of the aryl group as R² in the formula (I) can include aryl groups having 6 to 14 carbon atoms, such as phenyl, naphthyl, anthryl, and phenanthryl groups.

The aralkyl group as R² in the formula (I) has an aryl moiety, examples of which can be the same as those of the aryl group described above, and an alkyl moiety, examples of which can be the same as those of the alkyl group described above. Specific examples thereof can include aralkyl groups having 7 to 15 carbon atoms, such as benzyl, phenethyl, phenylpropyl, phenylbutyl, benzhydryl, trityl, naphthylmethyl, and naphthylethyl.

Among the ALAs, examples of the salts of the ALAs or the derivatives thereof can include pharmacologically acceptable acid-addition salts, metal salts, ammonium salts, and organic amine-addition salts. Examples of the acid-addition salt can include individual inorganic acid salts such as hydrochloride, hydrobromide, hydroiodide, phosphate, nitrate, and sulfate, and individual organic acid-addition salts such as formate, acetate, propionate, toluenesulfonate, succinate, oxalate, lactate, tartrate, glycolate, methanesulfonate, butyrate, valerate, citrate, fumarate, maleate, and malate. Examples of the metal salt can include individual alkali metal salts such as lithium salt, sodium salt, and potassium salt, individual alkaline earth metal salts such as magnesium and calcium salt, and individual metal salts such as aluminum and zinc. Examples of the ammonium salt can include ammonium salt and alkylammonium salts such as tetramethylammonium salt. Examples of the organic amine salt can include individual salts such as triethylamine salt, piperidine salt, morpholine salt, and toluidine salt.

Examples of esters of the ALAs can include, but are not limited to, methyl ester, ethyl ester, propyl ester, butyl ester, pentyl ester, hexyl ester, and heptyl ester.

Since the ALAs are substances having high safety, the administration route thereof to the subject is not limited and various administration routes can be selected. Examples thereof can include oral administration including sublingual administration, and parenteral administration such as inhalation administration, direct administration to a target tissue or organ through a catheter, intravenous administration including intravenous drip, transdermal administration using a patch or the like, suppositories, and administration by a forced enteral alimentation method using a nasogastric tube, a naso-intestinal tube, a gastrostomy tube, or an enteral feeding tube. The simplest administration method can be oral administration.

The dose of the ALAs to the subject can be determined as the optimum dose by those skilled in the art (e.g., physicians) according to the treatment stage, symptoms, etc. of the subject. Specifically, the dose can be 0.1 mg to 1000 mg/day, preferably 0.2 mg to 500 mg/day or 0.3 mg to 250 mg/day, more preferably 0.4 mg to 100 mg/day, most preferably 0.5 mg to 20 mg/day, per kg of body weight of the subject in terms of ALA.

In the present disclosure, the ALAs can be applied with an iron compound to the subject. Non-limiting examples of the iron compound can include ferrous citrate, sodium ferrous citrate (SFC), sodium iron citrate, ammonium iron citrate, ferric pyrophosphate, heme iron, dextran iron, iron lactate, ferrous gluconate, DTPA iron, sodium iron diethylenetriaminepentaacetate, ammonium iron diethylenetriaminepentaacetate, sodium iron ethylenediaminetetraacetate, ammonium iron ethylenediaminepentaacetate, triethylenetetramine iron, sodium iron dicarboxymethylglutamate, ammonium iron ammonium dicarboxymethylglutamate, lactoferrin iron, transferrin iron, ferric chloride, iron sesquioxide, sodium iron chlorophyllin, ferritin iron, ferrous fumarate, ferrous pyrophosphate, saccharated iron oxide, iron acetate, iron oxalate, ferrous succinate, sodium iron citrate succinate, iron sulfate, and iron glycine sulfide.

The terms used in the present specification are used for describing particular embodiments and do not intend to limit the invention, unless otherwise defined.

The term "comprising" used in the present specification means that described items (members, steps, factors, numbers, etc.) are present and the presence of the other items (members, steps, factors, numbers, etc.) is not excluded therefrom, unless the context evidently requires different interpretation.

All terms (including technical terms and scientific terms) used herein have the same meaning as that widely understood by those skilled in the technical field to which the present invention belongs, unless otherwise defined. The terms used herein should be interpreted as having consistent meaning with that in the present specification and related technical fields and should not be interpreted as having idealized or excessively formal meaning, unless different definitions are specified.

Hereinafter, the present invention will be described in more detail with reference to Examples. However, the present invention can be embodied in various forms and must not be interpreted as being limited to Examples described herein.

### [Examples]

Cells were isolated by trypsin and collagenase treatment of the heart harvested from a neonatal rat, and inoculated onto a petri dish. After culture for several days, the culture medium was replaced with a serum-free medium (DMEM), and 2 µM (final concentration) doxorubicin was added from 24 hours later in the serum-free medium, followed by recovery with a RIPA buffer 24 hours later. Aminolevulinic acid (5'-ALA, 1 mM) or ferrostatin-1 (Fer-1, 50 µM) was added 1 hour before doxorubicin addition. The recovered sample was used in the measurement of malondialdehyde (MDA) using thiobarbituric acid reactive substances (TBARS) assay kit (Cayman Chemical Company). The protein concentration of each sample was measured by the BCA method, and the MDA concentration was corrected with the protein concentration (nmol/mg protein) and normalized with a mean of the control group defined as 1.

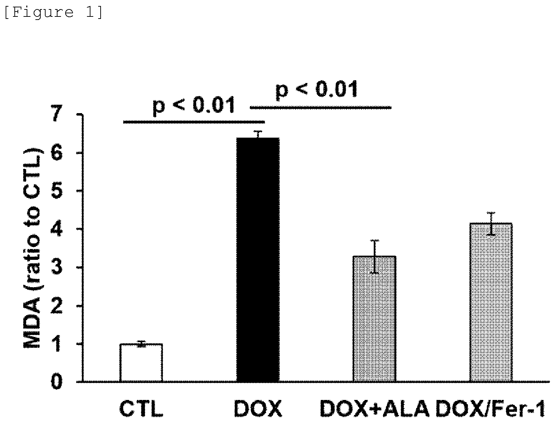

The test results are shown in Figure 1. As shown in the drawing, the addition of 5'-ALA suppressed increase in lipid peroxide level in heart cells ascribable to the influence of doxorubicin. The suppressive effect was equivalent to the effect of Fer-1 known as a ferroptosis inhibitor.

Cells were isolated by trypsin and collagenase treatment of the heart harvested from a neonatal rat, and inoculated onto a petri dish. After culture for several days, the culture medium was replaced with a serum-free medium (DMEM), and 2 µM (final concentration) doxorubicin was added from 24 hours later in the serum-free medium. Aminolevulinic acid (5'-ALA, 1 mM) or ferrostatin-1 (Fer-1, 50 µM) was added 1 hour before doxorubicin addition. Cell death evaluation assay was conducted from fluorescence intensity using Cell Counting Kit-F (Dojindo Laboratory) and a plate reader (Varioskan LUX Multimode Microplate Reader) 24 hours after doxorubicin addition. The obtained results were normalized with those of the control group defined as 1, and presented as data.

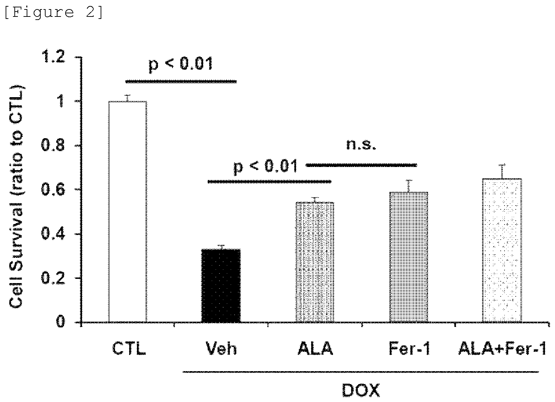

The test results are shown in Figure 2. As shown in the drawing, the addition of 5'-ALA suppressed the cell death (ferroptosis) of heart cells induced by doxorubicin. The suppressive effect was equivalent to the effect of Fer-1 known as a ferroptosis inhibitor.

Doxorubicin (6 mg/kg/dose) was administered a total of three times on days 0, 2, and 4 to the tail vein of an 8-week-old C57BL/6J mouse. For the administration of aminolevulinic acid, the aminolevulinic acid was dissolved in a drinking bottle so as to attain a dose of 300 mg/kg/day on the assumption that the daily water intake of the mouse was 4 ml/day. Drinking water administration was started from 3 days before doxorubicin administration. Measurement by echocardiography (Vevo 1100, FUJIFILM VisualSonics Inc.) was performed on 7 days and 14 days after the start of doxorubicin administration.

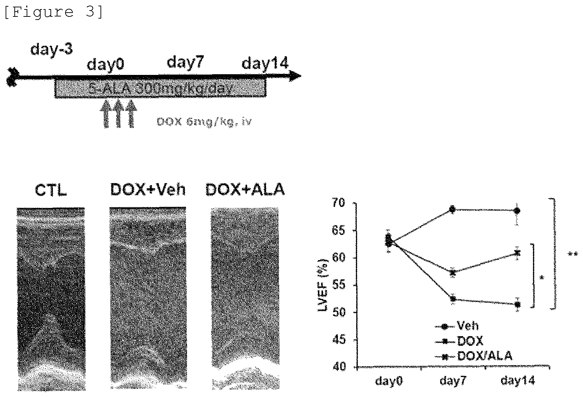

The test results are shown in Figure 3. As shown in the drawing, the prior administration of 5'-ALA significantly improved reduction in left ventricular systolic performance in the doxorubicin cardiomyopathy model.

After echocardiography on day 14, the mouse was euthanized, followed by the recovery of tissues, which were immediately cryopreserved in liquid nitrogen. Protein extraction was performed from the recovered frozen tissues using a RIPA buffer, and the extracted protein was immunostained with acrolein (JaICA, MAR-020n) by Western blot. The obtained image was quantitatively analyzed in image J (NIH). GAPDH (Santa Cruz Biotechnology Inc., sc-32233) was used as an internal control.

Likewise, protein extraction was performed from the recovered frozen tissues using a RIPA buffer, and the extracted sample was used in the measurement of malondialdehyde (MDA) using thiobarbituric acid reactive substances (TBARS) assay kit (Cayman Chemical Company). The protein concentration of each sample was measured by the BCA method, and the MDA concentration was corrected with the protein concentration (nmol/mg protein) and normalized with a mean of the control group defined as 1.

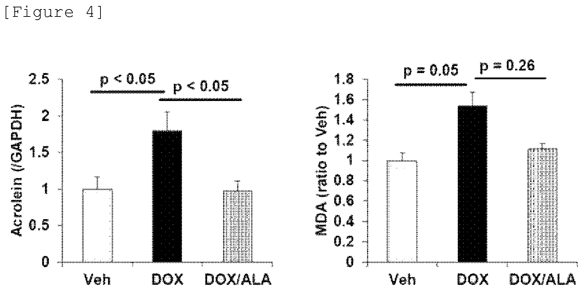

The test results are shown in Figure 4. As shown in the drawing, the prior administration of 5'-ALA significantly suppressed increase in lipid peroxide (acrolein and MDA) level in heart cells in the doxorubicin cardiomyopathy model.

Cells were isolated by trypsin and collagenase treatment of the heart harvested from a neonatal rat, and inoculated onto a petri dish. After culture for several days, the culture medium was replaced with a serum-free medium (DMEM), and doxorubicin (DOX; 1 or 2 µM), daunorubicin (DNR; 1 or 2 µM), pirarubicin (THP; 1 or 2 µM), idarubicin (IDR; 1 or 2 µM), or epirubicin (EPI; 1 or 2 µM) was added (final concentrations for all) from 24 hours later in the serum-free medium. Aminolevulinic acid (5'-ALA, 1 mM) was added 2 hours before doxorubicin addition. Cell death evaluation assay was conducted by fluorescence intensity measurement using Cell Counting Kit-F (Dojindo Laboratory) and a plate reader (Varioskan LUX Multimode Microplate Reader) 26 hours after doxorubicin addition. The obtained results were normalized with those of the control group defined as 1, and presented as data.

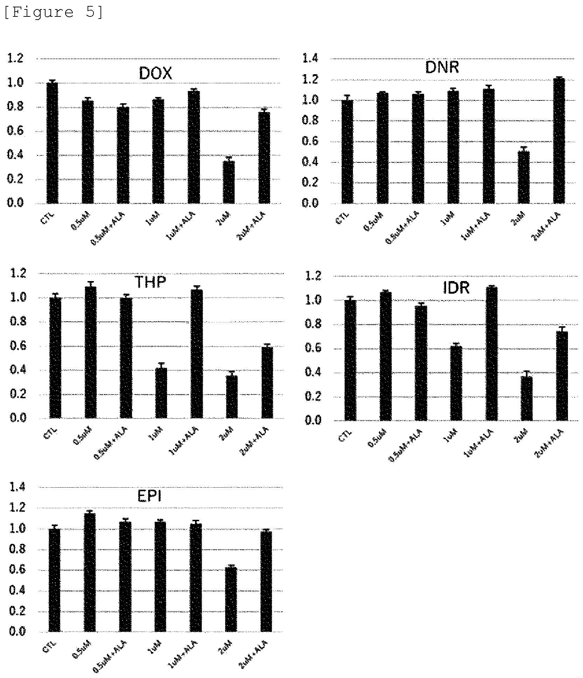

The test results are shown in Figure 5. As shown in the drawing, the cell death (ferroptosis) of heart cells was observed in association with increase in the dose of the anticancer agent in all the administration groups. However, the prior administration of 5'-ALA suppressed the cell death of heart cells in all the administration groups.

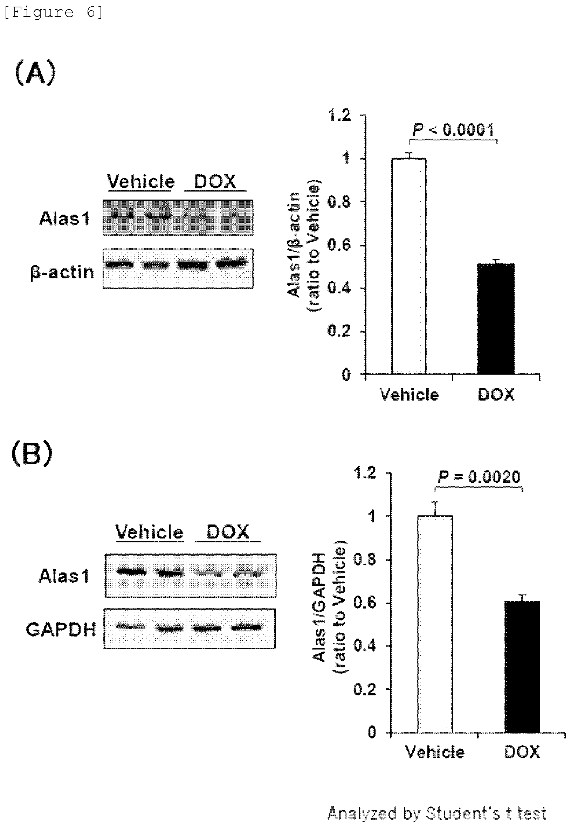

After doxorubicin (2 µM, 24 hours) addition to isolated rat primary cardiomyocytes, protein extraction was performed using a RIPA buffer, and the extracted protein was immunostained with Alas1 (Abeam plc, ab84962) by Western blot. The obtained image was quantitatively analyzed in image J (NIH). β-Actin (Santa Cruz Biotechnology Inc., sc-47778) was used as an internal control. The results are shown in Figure 6A.

Next, doxorubicin (6 mg/kg/dose) was administered a total of three times on days 0, 2, and 4 to the tail vein of an 8-week-old C57BL/6J mouse. On day 7, the mouse was euthanized, followed by the recovery of tissues, which were immediately cryopreserved in liquid nitrogen. Protein extraction was performed from the recovered frozen tissues of the heart using a RIPA buffer, and the extracted protein was immunostained with Alas1 (Abeam plc, ab84962) by Western blot. The obtained image was quantitatively analyzed in image J (NIH). GAPDH (Santa Cruz Biotechnology Inc., sc-32233) was used as an internal control. The results are shown in Figure 6B.

As shown in Figures 6A and 6B, it became evident that the level of ALAS1, a rate determining enzyme of a heme synthesis pathway in cardiomyocytes and cardiac muscular tissues, was decreased by the action of doxorubicin.

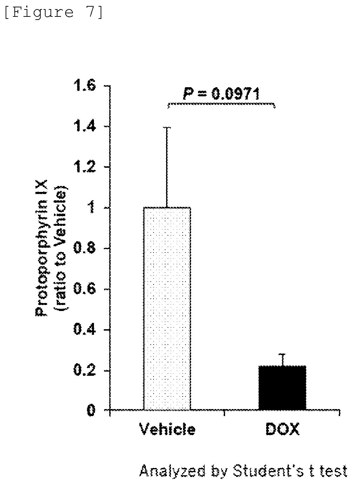

After doxorubicin (2 µM, 24 hours) addition to isolated rat primary cardiomyocytes, the cells were recovered with PBS. 50% acetic acid and a dimethylformamide (DMF)/2-propanol (IPA) mixed solution were added to the recovered sample, and the mixture was vigorously mixed by vortex. After centrifugation, a supernatant was recovered (supernatant 1), and a DMF/IPA mixed solution was added again to pellets, followed by centrifugation again. The supernatant thus obtained by centrifugation (supernatant 2) and the supernatant 1 were combined and used as protoporphyrin IX extracts. Acetonitrile was added to the extracts, and the mixture was deproteinized and then centrifuged at 20000 g for 20 minutes. Then, a supernatant was applied to a mass spectrometer (column: 1.8 µM, 2.1 × 100 mm; Waters, MA, USA, measurement equipment: Q Exactive Ultimate 3000; Thermo Fisher Scientific Inc.) to measure protoporphyrin IX (PPIX) in the cardiomyocytes. The results are shown in Figure 7.

As shown in Figure 7, it became evident that the level of protoporphyrin IX (PpIX), an intermediate metabolite of a heme synthesis pathway in cardiomyocytes, was decreased by the action of doxorubicin.

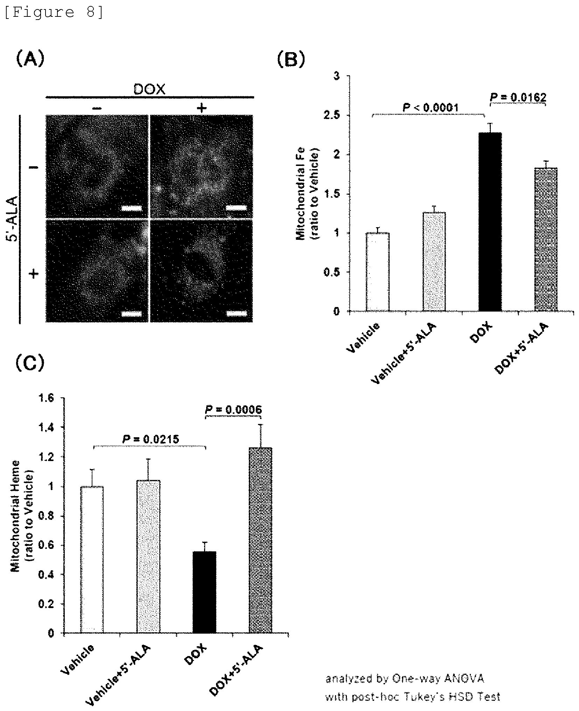

5'-ALA (1 mM) was added to isolated rat primary cardiomyocytes, and doxorubicin (2 µM) was added from 1 hour later. The cells were washed once with PBS 24 hours after doxorubicin addition and cultured for 30 minutes in a medium supplemented with Mito-FerroGreen (5 µM) for fluorescent staining of mitochondrial iron. The cells were washed with PBS again and then observed under a fluorescence microscope (BZ-X800, Keyence Corp.) (Figure 8A). The signal intensity of the obtained image was quantitatively analyzed in image J (NIH). The results are shown in Figure 8B.

As shown in Figures 8A and 8B, it became evident that although an iron excess state occurs in cardiomyocytes by the action of doxorubicin, the iron excess state is normalized by the action of 5'-ALA.

5'-ALA (1 mM) was added to isolated rat primary cardiomyocytes, and doxorubicin (2 µM) was added from 1 hour later. The cells were recovered with PBS 24 hours after doxorubicin addition, and mitochondria were isolated using mitochondria isolation kit (P507L, 101 Bio, LLC, Mountain View, CA, USA). Protein extraction was performed from the isolated mitochondria using a RIPA buffer, and mitochondrial heme was measured using QuantiChrom Heme Assay Kit (BioAssay Systems, Hayward, CA, USA). An alkali solution was added to the protein extraction solution, and absorbance at 400 nm was measured in a plate reader (Varioskan LUX Multimode Microplate Reader) 5 minutes later. The amount of heme was estimated from a calibration curve. The measured amount of heme was corrected by division by the protein concentration of the protein extracts. The results are shown in Figure 8C.

As shown in Figure 8C, it became evident that although a heme synthesis disorder occurs in cardiomyocytes by the action of doxorubicin, the disorder is normalized by the action of 5'-ALA.

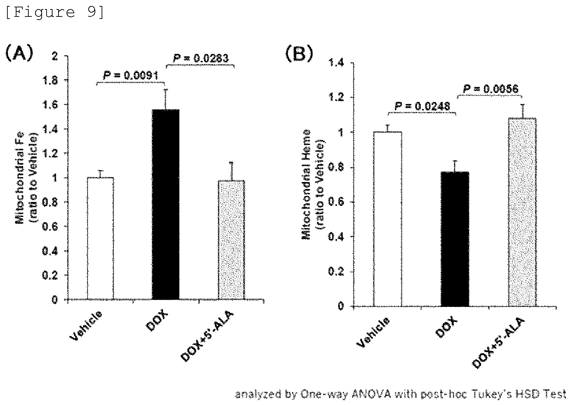

Doxorubicin (6 mg/kg/dose) was administered a total of three times on days 0, 2, and 4 to the tail vein of an 8-week-old C57BL/6J mouse. 5'-ALA was added to drinking water (dissolved at 1.875 mg/mL so as to attain an oral dose of 300 mg 5'-ALA/kg/day on the assumption that the water intake per mouse (25 g) was 4 ml/day) 3 days before initial doxorubicin administration and administered. On day 14, the mouse was euthanized, followed by the recovery of tissues, which were immediately cryopreserved in liquid nitrogen. Mitochondria were isolated from the recovered frozen tissues of the heart by the centrifugation method using a HES buffer, and protein extraction was performed using a RIPA buffer. The protein extracts were adjusted to pH 2 to 3 by the addition of HCl and centrifuged, and the obtained supernatant was subjected to the measurement of absorbance at 560 nm by the ferrozine method using Metallo Assay Kit (Metallo Assay Iron LS, Metallogenics, Chiba, Japan). The total amount of iron was estimated from a calibration curve. The total amount of iron was corrected by division by the protein concentration of the protein extracts. The results are shown in Figure 9A.

As shown in Figure 9A, it became evident that although an iron excess state occurs in cardiac muscular tissues by the action of doxorubicin, the iron excess state is normalized by the action of 5'-ALA.

Doxorubicin (6 mg/kg/dose) was administered a total of three times on days 0, 2, and 4 to the tail vein of an 8-week-old C57BL/6J mouse. 5'-ALA was added to drinking water (dissolved at 1.875 mg/mL so as to attain an oral dose of 300 mg 5'-ALA/kg/day on the assumption that the water intake per mouse (25 g) was 4 ml/day) 3 days before initial doxorubicin administration and administered. On day 14, the mouse was euthanized, followed by the recovery of tissues, which were immediately cryopreserved in liquid nitrogen. Mitochondria were isolated from the recovered frozen tissues of the heart by the centrifugation method using a HES buffer. Protein extraction was performed from the isolated mitochondria using a RIPA buffer, and mitochondrial heme was measured using QuantiChrom Heme Assay Kit (BioAssay Systems, Hayward, CA, USA). An alkali solution was added to the protein extracts, and absorbance at 400 nm was measured in a plate reader (Varioskan LUX Multimode Microplate Reader) 5 minutes later. The amount of heme was estimated from a calibration curve. The measured amount of heme was corrected by division by the protein concentration of the protein extracts.

As shown in Figure 9B, it became evident that although heme synthesis disorder occurs in cardiac muscular tissues by the action of doxorubicin, the disorder is normalized by the action of 5'-ALA.

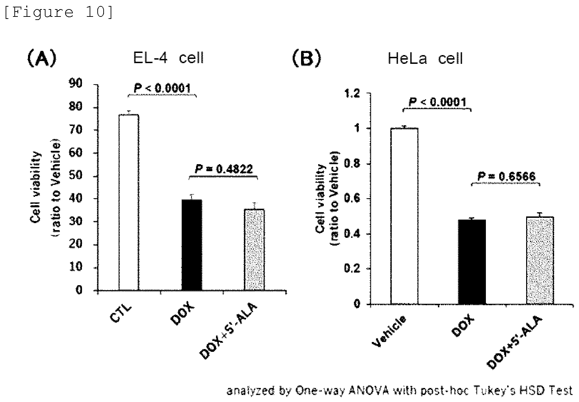

5'-ALA (1 mM) was added to EL-4 cells (mouse malignant lymphoma cells), and doxorubicin (1 µM) was added thereto 1 hour later. The cells were recovered 24 hours after doxorubicin addition, and dead cells were stained by Trypan Blue staining. The numbers of live cells and dead cells were counted in an automatic cell counter (Countess II FL, Thermo Fisher Scientific Inc.). Cell survival evaluation was conducted from the ratio (%) of the number of live cells to the total number of cells. The results are shown in Figure 10A.

5'-ALA (1 mM) was added to HeLa cells (human uterine cervical cancer cells), and doxorubicin (2 µM) was added thereto 1 hour later. Cell death evaluation assay was conducted by fluorescence intensity measurement (ex 490 nm/em 520 nm) using Cell Counting Kit-F (Dojindo Laboratory) and a plate reader (Varioskan LUX Multimode Microplate Reader) 24 hours after doxorubicin addition. The obtained results were normalized with those of the control group defined as 1, and presented as data.

As shown in Figures 10A and 10B, although a cell death-inducing effect, which is the drug efficacy of doxorubicin, was observed in the cancer cells (mouse malignant lymphoma EL-4 cells and human uterine cervical cancer HeLa cells), the effect was not attenuated by the application of 5'-ALA.

## Claims

1. A pharmaceutical composition for reducing toxicity of an anthracycline anticancer agent, comprising 5-aminolevulinic acids or derivatives thereof or salts of the acids or the derivatives as active ingredients.

2. The pharmaceutical composition according to claim 1, further comprising an iron compound.

3. The pharmaceutical composition according to claim 1 or 2, wherein the toxicity is depression of cardiac functions or elevation in lipid peroxide level.

4. The pharmaceutical composition according to any one of claims 1 to 3, wherein the pharmaceutical composition is orally administered.

5. The pharmaceutical composition according to any one of claims 1 to 4, wherein the pharmaceutical composition is prepared such that the 5-aminolevulinic acids or the derivatives thereof or the salts of the acids or the derivatives are administered at a dose of 0.5 mg/kg to 20 mg/kg to the subject.

6. The pharmaceutical composition according to any one of claims 1 to 5, wherein the pharmaceutical composition contains 5 mg to 1500 mg of the 5-aminolevulinic acids or the derivatives thereof or the salts of the acids or the derivatives.

7. The pharmaceutical composition according to any one of claims 1 to 6, wherein the pharmaceutical composition is administered to the subject concurrently with administration of the anthracycline anticancer agent or 2 hours to 7 days before the administration.

8. The pharmaceutical composition according to any one of claims 1 to 6, wherein the pharmaceutical composition is administered every day to the subject over a period from 1 day to 15 days before administration of the anthracycline anticancer agent.

9. The pharmaceutical composition according to any one of claims 1 to 6, wherein the pharmaceutical composition is continuously administered to the subject from at least 3 days (preferably 7 days) before administration of the anthracycline anticancer agent to at least 7 days (preferably 14 days) after the administration of the anthracycline anticancer agent.

10. The pharmaceutical composition according to claim 9, wherein the administration cycle is repeated every 1 week to 4 weeks.

11. The pharmaceutical composition according to any one of claims 1 to 10, wherein the 5-aminolevulinic acids are compounds represented by the following formula (I):
[Formula 1]
R¹-NHCH₂COCH₂CH₂COOR² (I)
wherein R¹ represents a hydrogen atom or an acyl group, and R² represents a hydrogen atom, a linear or branched alkyl group, a cycloalkyl group, an aryl group, or an aralkyl group,
or pharmacologically acceptable salts or esters thereof.

12. The pharmaceutical composition according to any one of claims 1 to 11, wherein the iron compound is one or two or more iron compounds selected from the group consisting of ferrous citrate, sodium ferrous citrate, sodium iron citrate, ammonium iron citrate, ferric pyrophosphate, heme iron, dextran iron, iron lactate, ferrous gluconate, DTPA iron, sodium iron diethylenetriaminepentaacetate, ammonium iron diethylenetriaminepentaacetate, sodium iron ethylenediaminetetraacetate, ammonium iron ethylenediaminepentaacetate, triethylenetetramine iron, sodium iron dicarboxymethylglutamate, ammonium iron ammonium dicarboxymethylglutamate, lactoferrin iron, transferrin iron, ferric chloride, iron sesquioxide, sodium iron chlorophyllin, ferritin iron, ferrous fumarate, ferrous pyrophosphate, saccharated iron oxide, iron acetate, iron oxalate, ferrous succinate, sodium iron citrate succinate, iron sulfate, and iron glycine sulfide.

13. The pharmaceutical composition according to any one of claims 1 to 12, wherein the anthracycline anticancer agent is an anticancer agent selected from the group consisting of doxorubicin, aclarubicin, pirarubicin, idarubicin, epirubicin, daunorubicin, amrubicin, their derivatives, and their pharmacologically acceptable salts.

14. A pharmaceutical composition for suppressing tumor growth, comprising an anthracycline anticancer agent as an active ingredient, wherein
the pharmaceutical composition is used with 5-aminolevulinic acids or derivatives thereof or salts of the acids or the derivatives.

15. The pharmaceutical composition according to claim 14, wherein the 5-aminolevulinic acids or the derivatives thereof or the salts of the acids or the derivatives are administered with an iron compound to the subject.

16. The pharmaceutical composition according to claim 14 or 15, wherein the 5-aminolevulinic acids or the derivatives thereof or the salts of the acids or the derivatives are administered for reducing toxicity of the anthracycline anticancer agent.

17. The pharmaceutical composition according to any one of claims 14 to 16, wherein the toxicity is depression of cardiac functions or elevation in lipid peroxide level.

18. The pharmaceutical composition according to any one of claims 14 to 17, wherein the 5-aminolevulinic acids or the derivatives thereof or the salts of the acids or the derivatives are orally administered.

19. The pharmaceutical composition according to any one of claims 14 to 18, wherein a therapeutically effective amount of the anthracycline anticancer agent is administered in combination with 0.5 mg/kg to 20 mg/kg of the 5-aminolevulinic acids or the derivatives thereof or the salts of the acids or the derivatives.

20. The pharmaceutical composition according to any one of claims 14 to 19, wherein the 5-aminolevulinic acids or the derivatives thereof or the salts of the acids or the derivatives are administered concurrently with administration of the anthracycline anticancer agent or 2 hours to 7 days before the administration.

21. The pharmaceutical composition according to any one of claims 14 to 19, wherein the 5-aminolevulinic acids or the derivatives thereof or the salts of the acids or the derivatives are administered every day over a period from 1 day to 15 days before (preferably 1 day to 3 days before) administration of the anthracycline anticancer agent.

22. The pharmaceutical composition according to any one of claims 14 to 19, wherein the 5-aminolevulinic acids or the derivatives thereof or the salts of the acids or the derivatives are continuously administered to the subject from at least 3 days (preferably 7 days) before administration of the anthracycline anticancer agent to at least 7 days (preferably 14 days) after the administration of the anthracycline anticancer agent.

23. The pharmaceutical composition according to claim 22, wherein the administration cycle is repeated every 1 week to 4 weeks.

24. The pharmaceutical composition according to any one of claims 14 to 23, wherein the 5-aminolevulinic acids are compounds represented by the following formula (1):
[Formula 2]
R¹-NHCH₂COCH₂CH₂COOR² (I)
wherein R¹ represents a hydrogen atom or an acyl group, and R² represents a hydrogen atom, a linear or branched alkyl group, a cycloalkyl group, an aryl group, or an aralkyl group, or pharmacologically acceptable salts or esters thereof.

25. The pharmaceutical composition according to any one of claims 14 to 24, wherein the iron compound is one or two or more iron compounds selected from the group consisting of ferrous citrate, sodium ferrous citrate, sodium iron citrate, ammonium iron citrate, ferric pyrophosphate, heme iron, dextran iron, iron lactate, ferrous gluconate, DTPA iron, sodium iron diethylenetriaminepentaacetate, ammonium iron diethylenetriaminepentaacetate, sodium iron ethylenediaminetetraacetate, ammonium iron ethylenediaminepentaacetate, triethylenetetramine iron, sodium iron dicarboxymethylglutamate, ammonium iron ammonium dicarboxymethylglutamate, lactoferrin iron, transferrin iron, ferric chloride, iron sesquioxide, sodium iron chlorophyllin, ferritin iron, ferrous fumarate, ferrous pyrophosphate, saccharated iron oxide, iron acetate, iron oxalate, ferrous succinate, sodium iron citrate succinate, iron sulfate, and iron glycine sulfide.

26. The pharmaceutical composition according to any one of claims 14 to 25, wherein the anthracycline anticancer agent is an anticancer agent selected from the group consisting of doxorubicin, aclarubicin, pirarubicin, idarubicin, epirubicin, daunorubicin, amrubicin, their derivatives, and their pharmacologically acceptable salts.

27. A method for reducing toxicity of an anthracycline anticancer agent in a subject, comprising the step of:
administering 5-aminolevulinic acids or derivatives thereof or salts of the acids or the derivatives to the subject concurrently with or separately from administration of the anthracycline anticancer agent to the subject.

28. A method for suppressing tumor growth in a subject, comprising the step of:
administering 5-aminolevulinic acids or derivatives thereof or salts of the acids or the derivatives and an anthracycline anticancer agent concurrently or separately to the subject.
